# EUROPÄISCHE PATENTSCHRIFT

(11) **EP 0 946 099 B1**
(45) Veröffentlichungstag und Bekanntmachung des Hinweises auf die Patenterteilung: **15.04.2015**
(21) Anmeldenummer: 97953783.4
(22) Anmeldetag: 11.12.1997
(51) Int. Cl.: A01N 43/90, A61K 31/70, A61K 31/365, A01N 61/00, A01N 57/24, A01N 51/00, A01N 47/44, A01N 47/40, A01N 43/86, A01N 43/78

(54) **ENDO-EKTO-PARASITIZIDE MITTEL**
ENDOPARASITICIDAL AND ECTOPARASITICIDAL AGENTS
AGENTS ENDOPARASITICIDES ET ECTOPARASITICIDES

(30) Priorität: 23.12.1996 DE 19654079
(43) Veröffentlichungstag der Anmeldung: 06.10.1999
(73) Patentinhaber: Bayer Intellectual Property GmbH, 40789 Monheim (DE)
(72) Erfinder: SIRINYAN, Kirkor, D-51467 Bergisch Gladbach (DE); DORN, Hubert, D-42115 Wuppertal (DE); HEESCHEN, Kerstin, D-51381 Leverkusen (DE); HEUKAMP, Ulrich, D-51515 Kürten (DE); KUJANEK, Richard, D-51061 Köln (DE)
(74) Vertreter: Gille Hrabal
(86) Internationale Anmeldenummer: PCT/EP1997/006926
(87) Internationale Veröffentlichungsnummer: WO 1998/027817

(56) Entgegenhaltungen:
- EP-A- 0 836 851
- WO-A-95/33453
- WO-A-96/38165
- WO-A1-96/17520
- DE-A- 19 519 007
- GB-A- 2 221 621
- US-A- 4 199 569
- ZA-A- 8 402 571
- CENTRAL PATENTS INDEX, BASIC ABSTRACTS JOURNAL Week 8609 23.April 1986 Derwent Publications Ltd., London, GB; AN 86-061936 XP002064421 & ZA 8 402 571 A (MERCK & CO INC) , 7.Oktober 1985
- I.D. HARROW & K.A.F. GRATION: "Mode of Action of the Anthelminitcs Morantel, Pyrantel and Levamisole on Muscle Cell Membrane of the Nematode Ascaris suum" PESTICIDE SCIENCE, Bd. 16, 1985, BARKING GB, Seiten 662-72, XP002064420
- WESLEY L. SHOOP ET AL: "Structure and activity of avermectins and milbemycins in animal health", VETERINARY PARASITOLOGY, vol. 59, no. 2, 1 September 1995 (1995-09-01), pages 139-156, XP055091744, ISSN: 0304-4017, DOI: 10.1016/0304-4017(94)00743-V

## Beschreibung

Die vorliegende Erfindung betrifft Mischungen von a) Avermectin B₁ₐ/B_{1b}, 22,23-Dihydroavermectin B₁ₐ/B_{1b}, Doramectin oder Moxidectin mit b) Imidacloprid, Ti 435 oder AKD 1022 sowie gegebenenfalls weiteren Wirkstoffen sowie Verdünnungsmitteln oder Trägerstoffen zur Herstellung von Mitteln zur dermalen Verabreichung der unter a) und b) genannten Wirkstoffe zur Bekämpfung von Ekto- und Endoparasiten in einer einzigen Behandlung.

Gastrointestinale Nematodeninfektionen von Hunden werden in den meisten Fällen von Spezies der drei Nemadodenfamilien Ascarididae, Ancylostomatidae und Trichuridae verursacht. In Katzen sind hauptsächlich die zwei Nematodenfamilien Ascarididae und Ancylostomatidae weltweit verbreitet. Nach Durchlaufen von verschiedenen Entwicklungsstadien in den unterschiedlichsten Geweben der Wirtstiere kommt es zu einer patenten Infektion des Gastrointestinaltraktes. Während der Präpatenz und Patenz der Infektion verursacht die Parasitose von Rund-, Haken- und Peitschenwürmern erhebliche Probleme speziell bei jungen, heranwachsenden Hunden, Katzen und auch beim Menschen. Eine Therapie oder prophylaktische Behandlung ist daher dringend erforderlich, sowohl zur Heilung bereits erkrankter Tiere als auch zur Gesunderhaltung noch nicht infizierter Tiere.

Der Schutz vor Infektion bei Hund und Katze ist somit als Prophylaxe gegen Humaninfektionen, speziell bei Kindern, sehr bedeutungsvoll.

Besonders zu erwähnen ist der Parasit Dirofilaria immitis - eine Filarie, die endemisch in Teilen von Nord- bis Südamerika, Afrika, Asien aber auch Australien verbreitet ist. Sie verursacht die bedeutende kanine- und feline kardiovaskuläre Dirofilariose. Die während der Dirofilaria immitis Infektion von Hunden und Katzen auftretenden schweren pathophysiologischen Veränderungen innerhalb des kardiovaskulären Systems, können einen dramatischen Krankheitsverlauf im Wirtstier bewirken.

Die Anthelminthika Ivermectin / Milbemycin aus der Klasse der makrocyclischen Lactone zeigen Wirkung gegen Dirofilaria immitis in Hund und Katze. Die Anwendung dieser Wirkstoffe erfolgt üblicherweise oral oder parenteral.

Der Flohbefall von Haustieren wie Hund und Katze ist für das befallene Tier nicht nur lästig, sondern fügt den befallenen Tieren auch erhebliche Schmerzen (Stichverletzungen, Juckreiz und Allergien) und Schäden (Blutverlust) zu. Flöhe können außerdem verschiedene Bandwurmarten übertragen. Sie stellen daher auch ein medizinisches Problem für die befallenen Tiere sowie für die Tierhalter dar. Auch der Tierhalter kann von Flöhen befallen werden. Bei manchen Menschen führt dies zur Flohstichallergie. Eine wirksame Bekämpfung der Flöhe bei Hunden und Katzen war deshalb von jeher wünschenswert und notwendig, zumal diese Haustiere in steigender Zahl und zunehmend engerem Kontakt mit den Menschen leben.

Es sind bislang zahlreiche insektizide Wirkstoffe zur Bekämpfung von Flöhen bekannt geworden. Solche Wirkstoffe sind z.B. aus der Klasse der Carbamate, Propoxur, Bendiocarb, Carbaryl, aus der Klasse der Phosphorsäureester, Fenthion, Diazinon, aus der Klasse der Pyrethroide, Permethrin, Cypermethrin, Resmethrin.

Bei diesen Wirkstoffen handelt es sich um Kontaktinsektizide mit überwiegender Wirkung auf die adulten Flöhe, die dermal verabreicht werden.

Sollen Haustiere gegen beide Probleme geschützt werden, waren bisher zwei getrennte Behandlungen üblich (parenterale oder orale Behandlung gegen Endoparasiten, dermale Behandlung gegen Ektoparasiten). Es war wünschenswert, diese beiden Behandlungen durch eine einzige Behandlung zu ersetzen.

Kombinationsprodukte, üblicherweise zur Erweiterung des Wirkungsspektrums beim Einsatz gegen Endoparasiten, waren schon bekannt.

Eine kombinierte Verabreichung von Endoparasitiziden und Ektoparasitiziden war bisher in der Praxis unüblich.

DE 195 19 007 A1 betrifft Chlornicotinyl-Insektizide, und nennt unter anderen auch Imidacloprid. In Mischung mit Synergisten aus einer umfassenden Gruppe verschiedener Verbindungen, worin unter zahlreichen anderen auch Abamectin (Avermectin) als möglicher Mischungspartner genannt wird. Allerdings wird in der Offenlegungsschrift lediglich eine Anwendung im Pflanzenschutz erwähnt..

Gegenstand der GB 222 16 21 A sind synergistische Kombinationen von Avermectinen mit Pyrantel oder Pyrantelpamoat.

ZA 8402571 A betrifft Wirkstoffkombinationen von Avermectinen mit z.B. Coumaphos oder Carbaryl, die jedoch weder topisch noch dermal verabreicht werden.

Gegenstand der WO 96/38165 A sind endoparasitizide Mittel auf Basis von Mischungen von Avermectinen, 22,23-Dihydroyvermectinen B1 und Milbemycinen mit cyclischen Depsipeptiden sowie gegebenenfalls den Endoparasitiziden Praziquantel oder Epsiprantel. 04 beschreibt damit ausschließlich synergistische Effekte derartiger Mischungen in Bezug auf Endoparasiten.

WO 95/33453 A betrifft Verbindungen der Agonisten und Antagonisten der nicotinergen Acetycholinrezeptoren von insokten, und offenbart deren Wirkung und Verwendung zur Bekämpfung von Endoparasiten.

Die ältere, jedoch nicht vorveröffentlichte europäische Patentanmeldung EP 0 836 851 A betrifft Amidinverbindungen zur Verwendung in der Bekämpfung von Krankheiten, die durch Ekto- und Endoparasiten ausgelöst werden.

Die vorliegende Erfindung betrifft die Verwendung von a) Avermectin B₁ₐ/B_{1b}, 22,23-Dihydroavermectin B₁ₐ/B_{1b}, Doramectin oder Moxidectin mit b) imidacloprid, Ti 435 oder AKD 1022 sowie gegebenenfalls weiteren Wirkstoffen sowie Verdünnungsmitteln oder Trägerstoffen zur Herstellung von Mitteln zur dermalen Verabreichung der unter a) und b) genannten Wirkstoffe zur Bekämpfung von Ekto- und Endoparasiten in einer einzigen Behandlung.

Es war dabei überraschend, daß sich diese Wirkstoffe, die aus völlig verschiedenen chemischen Klassen stammen sowie völlig unterschiedliche biologische Wirkungen zeigen, synergistisch beeinflussen.

Der Einsatz von Avermectinen, 22,23 Dihydroavermectinen B₁ (Ivermectinen) und Milbemycinen aus der Klasse der makrocyclischen Lactone als Endoparasitizide ist lange bekannt und Gegenstand zahlreicher Patentanmeldungen sowie Übersichtsartikel (z. B. Biologische Wirkungen in: "Ivermectin and Abamectin" W. C. Campbell, Ed., Springer Verlag, New York, N. Y., 1989; "Avermectins and Milbemycins Part II" H. G. Davies et al. Chem. Soc. Rev. 20 (1991) S. 271-339; Chemische Modifikationen in: G. Lukacs et al. (Eds.), Springer-Verlag, New York, (1990), Chapter 3; Cydectin ™ [Moxidectin und Derivate]: G. T. Carter ct al. J. Chem. Soc. Chem. Com-mun. (1987), S. 402-404); EP 423 445-A1). Der Einsatz von Doramectin (Pfizer) als Endoparasitizid ist ebenso bekannt (vgl. "Doramectin - a potent novel endectozide" A. C. Goudie et al. Vet. Parasitol 49 (1993), S. 5-15).

Ferner sind Kombinationen von Avermectinen, 22,23-Dihydroavermectinen B₁ (Iver-mectinen) oder Milbemycinen mit bestimmten Anthelminthikaklassen, wie beispielsweise Benzimidazolen, Salizylamiden, Levamisol, Pyrantel oder Praziquantel Gegenstand zahlreicher Patentanmeldungen (z. B.: GB 2 252 730; GB 2 224 933; GB 2 21 3 722; EP-OS 59 074).

Als Avermectine und deren Derivate seien genannt Stoffgemische von makroliden Lactonen der allgemeinen Formel (I) 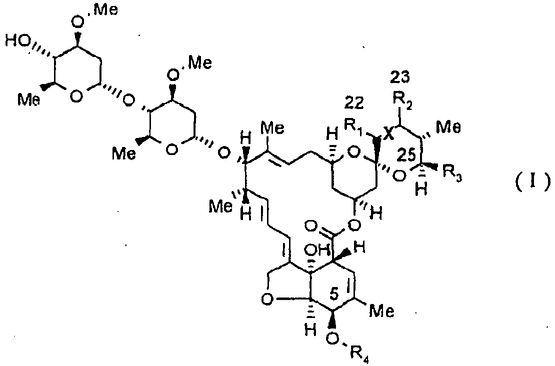 in welcher
die Reste R¹ bis R⁴ die in der nachfolgenden Tabelle 1 angegebene Bedeutung haben und X für eine Einfach- oder Doppelbindung zwischen der C₂₂- und C₂₃-Po-sition (-C₂₂R¹-X-C₂₃R²-) stehen kann.

Im Falle einer Doppelbindung befinden sich keine Substituenten (R¹, R²) an der C₂₂- und C₂₃-Position.

**Tabelle 1**

| **Makrocyclisches Lacton** | **-C₂₂R¹-X-C₂₃R²-** | **R³** | **R⁴** |
|---|---|---|---|
| Avermectine B₁ₐ | -CH=CH- | -sec-Bu | -H |
| Avermectin B_{1b} | -CH=CH- | -iso-Pr | -H |
| 22,23-Dihydroavermectin B₁ₐ | -CH₂-CH₂- | -sec-Bu | -H |
| 22,23-Dihydroavermectin B_{1b} | -CH₂-CH₂- | -iso-Pr | -H |
| Doramectin | -CH=CH- | -Chx | -H |

| | | | |
|---|---|---|---|
| 22,23-Dihydroavermectin B₁ steht für Ivermectin B₁; sec-Bu = sekundär Butyl; iso-Pr = Isopropyl; Chx = Cyclohexyl; | | | |

Die Avermectine und 22,23-Dihydroavermectine B₁ (Ivermectine) der allgemeinen Formel (I) werden in der Regel als Gemische eingesetzt. Von besonderem Interesse ist hierbei das Produkt Abamectin, das im wesentlichen die Avermectine B₁ enthält, und deren Hydrierungsprodukte die 22,23-Dihydröavermectine B₁ (Ivermectin).

Die mit "b" bezeichneten Verbindungen der makrocyclischen Lactone, die in der C₂₅-Position einen iso-Propylrest besitzen, müssen nicht notwendigerweise von den "a" Verbindungen, welche eine sec-Butylgruppe in der C₂₅-Position haben, getrennt werden. Es wird generell das Gemisch beider Substanzen, bestehend aus > 80 % sec-Butylderivat (B₁ₐ) und < 20 % iso-Propylderivat (B_{1b}) isoliert, und kann erfindungsgemäß verwendet werden. Zudem können bei den Stereoisomeren die Substituenten in der C₁₃- und C₂₃-Position sowohl α- als auch ß-ständig am Ringsystem angeordnet sein, d. h. sich oberhalb oder unterhalb der Molekülebene befinden.

Die Milbemycine haben die gleiche makrolide Ringstruktur wie Avermectine oder 22,23-Dihydroavermectine B₁ (Ivermectine), tragen aber keinen Substituenten (d.h. fehlendes Oleandrose-Disaccharidfragment) in Position 13 (R⁵ = Wasserstoff).

Als Milbemycin aus der Klasse der makrocyclischen Lactone sei Moxidectin gemäß der nachfolgenden Strukturformel mit der entsprechenden Restedefinition in Tabelle 2 genannt: 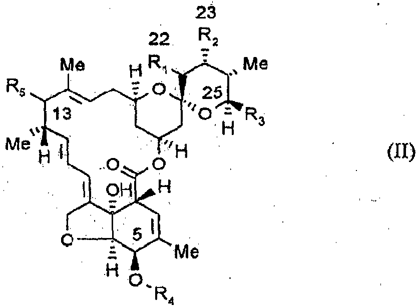 in welcher
die Reste R¹ bis R⁵ die in der nachfolgenden Tabelle 2 angegebene Bedeutung haben:

**Tabelle 2**

| **Makrocyclisches Lacton** | **R¹** | **R²** | **R³** | **R⁴** | **R⁵** |
|---|---|---|---|---|---|
| Moxidectin | -H | =N-O-Me | 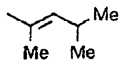 | -H | -H |

| | | | | | |
|---|---|---|---|---|---|
| Me = Methyl | | | | | |

Kombinationspartner in den erfindungsgemäßen Mischungen sind:
Avermectin B₁ₐ/B_{1b};
22,23-Dihydroavermectin B₁ₐ/B_{1b} (bzw. Ivermectin B₁ₐ/B_{1b});
Doramectin;
Moxidectin.

Agonisten oder Antanogisten der nicotinogen Acetylcholinrezeptoren von Insekten sind bekannt z.B. aus Europäische Offenlegungsschriften Nr. 464 830, 428 941, 425 978, 386 565, 383 091, 375 907, 364 844, 315 826, 259 738, 254 859, 235 725, 212 600, 192 060, 163 855, 154 178, 136 636, 303 570, 302 833, 306 696, 189 972, 455 000, 135 956, 471 372, 302 389; Deutsche Offenlegungsschriften Nr. 3 639 877, 3 712 307; Japanische Offenlegungsschriften Nr. 03 220 176, 02 207 083, 63 307 857, 63 287 764, 03 246 283, 04 9371, 03 279 359, 03 255 072; US-Patentschriften Nr. 5 034 524, 4 948 798, 4 918 086, 5 039 686, 5 034 404; PCT-Anmeldungen Nr. WO 91/17 659, 91/4965; Französische Anmeldung Nr. 2 611 114; Brasilianische Anmeldung Nr. 88 03 621.

Mischungspartner der vorgenannten Wirkstoffe Avermectin B₁ₐ/B_{1b}, 22,23,Dihydroavermectin B₁ₐ/B_{1b}. Doramectin oder Moxidectin sind die folgenden Verbindungen dieser Wirkstoffklasse Imidacloprid. Ti 435 oder AKD 1022 mit den nachstehenden Strukturformeln:

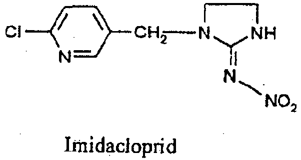

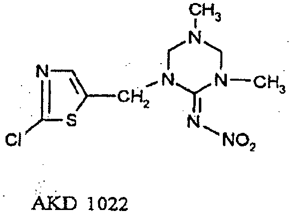

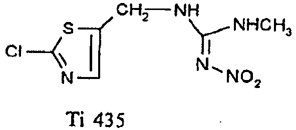

Zum Beispiel werden als erfindungsgemäße Kombinationspartner die 22, 23-Dihydroavermectine B₁ₐ/B_{1b} (Ivermectine B₁ₐ/B_{1b}) der allgemeinen Formel (Ia) aus der Klasse der makrocyclischen Lactone 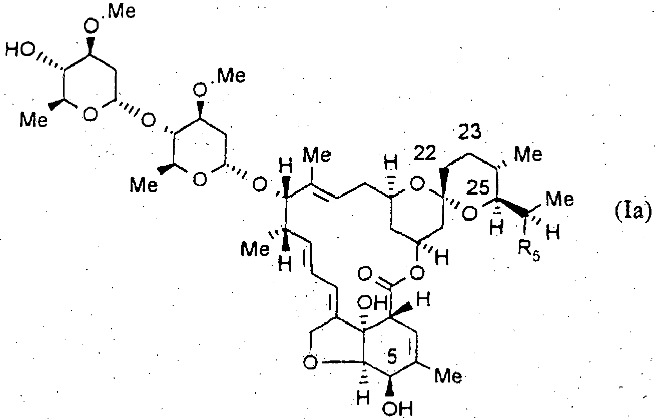 in welcher
- R⁵: für Methyl und Ethyl steht,
mit Imidacloprid gegebenenfalls in Gegenwart weiterer Wirkstoffe sowie Trägerstoffe in einem syner-gistisch wirkenden Verhältnis miteinander kombiniert.

Zum Beispiel werden als erfindungsgemäße Kombinationspartner die 22,23-Dihydroavermectine B₁ₐ/B_{1b} (Ivermectine B₁ₐ/B_{1b}) der allgemeinen Formel (Ia) aus der Klasse der makrocyclischen Lactone 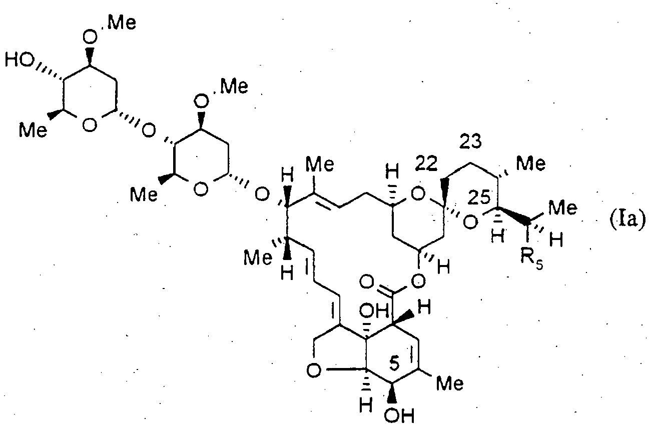 in welcher
- R⁵: für Methyl und Ethyl steht,
mit Ti 435 (Clothianidin) gegebenenfalls in Gegenwart weiterer Wirkstoffe oder Trägerstoffe in einem synergistisch wirkenden Verhältnis miteinander kombiniert.

Die endöparasitizide Wirksamkeit der erfindungsgemaßen Wirkstoffkombinationen liegt deutlich höher als von den Wirkungen der Einzelkomponenten zu erwarten war. Durch Anwendung dieser Kombinationen können daher die Aufwandmenge sowie die Zahl der Anwendungen reduziert werden.

Die erfindungsgemäßen Mittel eignen sich bei günstiger Warmblütertoxizität zur Bekämpfung von pathogenen Endoparasiten und Ektoparasiten, die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schadlinge sowie gegen resistente und normal sensible Arten wirksam. Durch die Bekämpfung der pathogenen Endoparasiten sollen Krankheit, Todesfälle und Leistungsminderungen vermindert werden, so daß durch den Einsatz der Wirkstoffe eine wirtschaftlichere und einfachere Tierhaltung möglich ist. Zu den pathogenen Endoparasiten zählen Nematoden, Acantocephalen insbesondere

Aus der Unterklasse der Monogenea z.B.: Gyrodactylus spp., Dactylogyrus spp., Polystoma spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp.,

Globocephalus spp., Syngamus spp., Cyathostoma spp., Metastrongylus spp., Dictyocaulus spp., Muellerius spp., protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.. Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Bei den Ektoparasiten seien genannt:
Aus der Ordnung der Anoplura z.B. Haematopinus spp., Linognathus spp., Solenopotes spp., Pediculus spp., Pthirus spp.;
aus der Ordnung der Mallophaga z.B. Trimenopon spp., Menopon spp., Eomenacanthus spp., Menacanthus spp., Trichodectes spp., Felicola spp., Damalinea spp., Bovicola spp;
aus der Ordnung der Diptera z.B. Chrysops spp., Tabanus spp., Musca spp., Hydrotaea spp., Muscina spp., Haematobosca spp., Haematobia spp., Stomoxys spp., Fannia spp., Glossina spp., Lucilia spp., Calliphora spp., Auchmeromyia spp., Cordylobia spp., Cochliomyia spp., Chrysomyia spp., Sarcophaga spp., Wohlfartia spp., Gasterophilus spp., Oesteromyia spp., Oedemagena spp., Hypoderma spp., Oestrus spp., Rhinoestrus spp., Melophagus spp., Hippobosca spp..

Aus der Ordnung der Siphonaptera z.B. Ctenocephalides spp., Echidnophaga spp., Ceratophyllus spp..

Besonders hervorgehoben sei die Wirkung gegen Siphonaptera, insbesondere gegen Flöhe.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Ganse. Puten, Enten, Strauße, Süß- und Salzwasserfische wie z.B. Forellen, Karpfen. Aale.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen

Die Anwendung der Wirkstoffe erfolgt direkt oder in Form von geeigneten Zubereitungen dermal.

Die dermale Anwendung geschieht z.B. in Form des Sprühens (Sprayen) oder Aufgießens (pour-on and spot-on).

Geeignete Zubereitungen sind:
Lösungen zum Gebrauch auf der Haut Aufgußformulierungen, Gele;
Emulsionen und Suspension zur dermalen Anwendung, halbfeste Zubereitungen;
Formulierungen bei denen der Wirkstoff in einer Salbengrundlage oder in einer Öl in Wasser oder Wasser in Öl Emulsionsgrundlage verarbeitet ist;
Feste Zubereitungen wie wirkstoffhaltige Formkorper.

Als Lösungsmittel seien genannt: Physiologisch verträgliche Lösungsmittel wie Wasser, Alkohole wie Ethanol, Butanol, Benzylalkohol, Glycerin, Propylenglykol, Polyethylenglykole, N-Methyl-pyrrolidon, 2-Pyrrolidon sowie Gemische dei selben.

Als Lösungsvermittler seien genannt. Lösungsmittel, die die Losung des Wirkstoffs im Hauptlosungsmittel fördern oder sein Ausfallen verhindern. Beispiele sind Polyvinylpyrrolidon, Polyvinylalkohol, polyoxyethyliertes Rhizinusol, polyoxyethylierte Sorbitanester.

Konservierungsmittel sind: Benzylalkohol, Trichlorbutanol, p-Hydroxybenzoesäureester, n-Butanol.

Lösungen zum Gebrauch auf der Haut werden aufgeträufelt, aufgestrichen, eingerieben, aufgespritzt oder aufgesprüht.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Gele werden auf die Haut aufgetragen oder aufgestrichen. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, cyclische Carbonate wie Propylencarbonat, Ethylencarbonat, aromatische und/oder aliphatische Kohlenwasserstoffe, pflanzliche oder synthetische Ole, DMF, Dimethylacetamid, n-Alkylpyrrolidone wie n-Methylpyrrolidon, n-Butyl- oder n-Octylpyrrolidon, N-Methylpyrrolidon, 2-Pyrrolidon, 2,2-Dimethyl-4-oxy-methylen-1,3-dioxolan und Glycerinformal..

Farbstoffe sind alle zur Anwendung am Tier zugelassenen Farbstoffe, die gelöst oder suspendiert sein können.

Resorptionsfordernde Stoffe sind z.B. DMSO, spreitende Öle wie Isopropylmyristat, Dipropylenglykolpelargonat, Silikonöle, bzw. deren Copolymerisate mit Polyethern, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsaure.

Haftmittel sind z.B. Cellulosederivate, Stärkederivate, Polyacrylate, natürliche Polymere wie Alginate, Gelatine.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Losungsmittel der anderen Phase homogenisiert

Als hydrophobe Phase (Ole) seien genannt: Paraffinöle, Silikonöle, natürliche Pflanzenöle wie Sesamöl, Mandelöl, Rizinusöl, synthetische Triglyceride wie Capryl/ Caprinsäure-biglycerid, Triglyceridgemisch mit Pflanzenfettsauren der Kettenlänge C₈₋₁₂ oder anderen speziell ausgewählten natürlichen Fettsauren, Partialglyceridgemische gesättigter oder ungesättigter eventuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsaurehexylester, Dipropylen-glykolpelargonat, Ester einer verzweigten Fettsäure mittlerer Kettenlange mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsaurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-ß-iminodipropionat oder Lecithin,
anionaktive Tenside, wie Na-Laurylsulfat, Fettalkoholethersulfate. Mono/Dialkylpolyglykoletherorthophosphorsaureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsaureanhydrid, Polyethylenglykole, Wachse, kolloidale Kieselsäure oder Gemische der aufgeführten Stoffe.

Suspensionen werden dermal angewendet. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen werden dermal verabreicht. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewunschte Form gebracht.

Die Wirkstoffe können in den Zubereitungen auch in Mischung mit Synergisten oder mit anderen Wirkstoffe, die gegen pathogene Endoparasiten wirken, vorliegen. Solche Wirkstoffe sind z.B. L-2,3,5,6-Tetrahydro-6-phenyl-imidazothiazol, Benzimidazolcarbamate, Pyrantel, Praziquantel, Epsiprantel.

Anwendungsfertige Zubereitungen enthalten die Wirkstoffe gegen Ektoparasiten in Konzentrationen von 10 ppm - 20 Gewichtsprozent, bevorzugt von 0,1-12,5 Gewichtsprozent.

Zubereitungen die vor Anwendung verdünnt werden, enthalten die Wirkstoffe gegen Ektoparasiten in Konzentrationen von 0,5-90 Gew.-%, bevorzugt von 5 bis 50 Gewichtsprozent.

Ferner enthalten die Zubereitungen die beschriebenen Wirkstoffe gegen Endoparasiten in Konzentration von 10 ppm - 2 Gew.-%, bevorzugt von 0,05-0,9 Gew.-%, ganz besonders bevorzugt 0,005 - 0,25Gew.-%.

In den erfindungsgemäßen Mitteln wird im Falle einer Anwendung im Hobbytier Hund im allgemeinen ein Gewichtsverhältnis von makrocyclischem Lacton zu Agonist oder Antagonist der nicotinergen Acetylcholinrezeptoren von Insekten wie 1 zu 500 bis 1000 bevorzugt 1 zu 500 bis 850 ganz besonders bevorzugt 1 zu 500 eingehalten.

Schließlich wird in den erfindungsgemäßen Mitteln im Falle einer Anwendung im Nutztier im allgemeinen ein Gewichtsverhältnis von makrocyclischem Lacton zu Agonist oder Antagonist der nicotinergen Acetylcholinrezeptoren von Insekten wie 1 zu 20 bis 400 bevorzugt 1 zu 20 bis 250 ganz besonder bevorzugt 1 zu 20 bis 50 eingehalten.

In den folgenden Beispielen wird als Agonist oder Antagonist der nicotinergen Acetylcholinrezeptoren von Insekten 1-[(6-Chlor-3-pyridinyl)methyl]-N-nitro-2-imidazolidinium (common name Imidacloprid) und als makrocyclisches Lacton Ivermectin eingesetzt.

### Beispiele

### Beispiel 1

### SL-(wasserlösliche Konzentrat)-Formulierung

| | |
|---|---|
| 18,3 g | Imidacloprid |
| 0,2 g | Ivermectin |
| 2,5 g | neutraler Emulgator auf Basis Alkylarylpolyglykolether |
| 3,5 g | Natriumsulfobernsteinsaurediisooctylester |
| 38,4 g | Dimethylsulfoxid und |
| 37,5 g | 2-Propanol |

### Beispiel 2

### pour-on-Formulierung

| | |
|---|---|
| 20,3 g | Imidacloprid |
| 0,2 g | Ivermectin |
| 1,8 g | Polyvinylalkohol |
| 1,8 g | Blockcopolymerisat auf Basis Ethylenoxid und Propylenoxid |
| 0,26 g | Xanthan Gum |
| 9,0 g | Glycerin |
| 59,2 g | destilliertes Wasser |

### Beispiel 3

### Spot-on Formulierung

| | |
|---|---|
| 10,000 g | Imidacloprid |
| 0,006 g | Ivermectin |
| 83,394 g | Benzylalkohol |
| 16,300 g | Propylencarbonat |
| 0,100 g | BHT (Buthydroxytoluol) |

### Beispiel 4

### Spot-on Formulierung

| | |
|---|---|
| 10,000 g | Imidacloprid |
| 0,050 g | Ivermectin |
| 83,350 g | Benzylalkohol |
| 16,300 g | Propylencarbonat |
| 0,100 g | BHT |

### Beispiel 5

### Spot-on Formulierung

| | |
|---|---|
| 10,000 g | Imidacloprid |
| 0,200 g | Ivermectin |
| 83,200 g | Benzylalkohol |
| 16,300 g | Propylencarbonat |
| 0,100 g | BHT |

### Anwendungsbeispiel A

1 ml der im Beispiel 1 angegebenen SL-Formulierung wurde als Lösung pour-on auf die Schulter eines mit 200 Flöhen infestierten Hundes aufgetragen. Das Versuchstier konnte sofort von adulten Flöhen befreit werden. Die erfindungsgemäße Behandlung führt zu einer 100 %igen Mortalitatsrate der Flöhe.

### Anwendungsbeispiel B

1 ml der in Beispiel 1 beschriebenen Formulierung wurde in 11 Wasser verdünnt und mit dieser Lösung -20 kg Hunde die mit Flöhen infestiert waren tropfnaß begossen. Es wurden folgende Ergebnisse erhalten:

**Tabelle B**

| Zeitraum Tag | Anzahl der Flöhe pro Hund | | % Wirkung |
|---|---|---|---|
| | unbehandelt | behandelt | |
| -1 Infestation mit 100 Flöhen | | | |
| 0 Behandlung und Zählung | 30 | 0 | 100 |
| 5, 8 Infestation mit 100 Flöhen | | | |
| 9 Zählung | 56 | 0 | 100 |
| 15 Infestation mit 100 Flöhen | | | |
| 16 Zählung | 76 | 0 | 100 |
| 19 Infestation mit 100 Flöhen (unbehandeltes Tiere) 250 Flöhen (behandelte Tiere | | | |
| 20 Zählung | 39 | 0 | 100 |
| 26 Infestation mit 100 Flöhen | | | |
| 27 Zählung | 43 | 0 | 100 |

### Anwendungsbeispiel C (nicht erfindungsgemäß)

### In-vivo Ncmatodentest

### Nematospiroides dubius in der Maus

Mäuse wurden experimentell mit Nematoden der Art Nematospiroides dubius infiziert. Zur Infektion wurde den Mäusen Nematospiroides dubius oral als 60 filariforme Larven appliziert.

Nach Ablauf der Präpatenzzeit wurden die suspendierten Wirkstoffe gemäß Beispiel 2 oral am 12. Tag nach der Infektion appliziert.

### Bestimmung der Wirksamkeit:

Die Selektion der Mäuse erfolgt am 20. Tag nach der Infektion. Die Auszählung der adulten Parasiten im *Duodenum* wird mittels Kompressorium durchgeführt. Der Behandlungserfolg in der Dosisgruppe wird ins Verhältnis zur unbehandelten Kontrollgruppe gesetzt.

In den nachfolgenden Tabellen A und B wird die Wirkung der Kombination gegen Nematospiroides dubius in der Maus angegeben.

**Tabelle C Wirkung der Kombination von Imidacloprid und Ivermectin B₁ₐ/B_{1b} gegen Nematospiroides dubius in der Maus nach oraler Applikation**

| **Wirkstoff und Menge [mg/kg]** | | **Reduktionsrate [%]** |
|---|---|---|
| Imidacloprid | 50,0 | 0 |
| Ivermectin B₁ₐ/B_{1b} | 0,1 | 0 |
| Ivermectin B₁ₐ/B_{1b} + Imidacloprid | 50,0 | |
| | 0,1 | 100 |
| Imidacloprid | 25,0 | 0 |
| Imidacloprid + Ivermectin B₁ₐ/B_{1b} | 25,0 | |
| | 0,1 | >80 |

### Beispiel D (nicht erfindungsgemäß)

### In-vivo Nematodentest

### Heterakis spumosa in der Maus

Mäuse wurden experimentell mit Nematoden der Art Heterakis spumosa infiziert. Zur Infektion wurde den Mäusen Heterakis spumosa oral als 90 embryonierte Eier appliziert.

Nach Ablauf der Präpatenzzeit wurden die suspendierten Wirkstoffe gemäß Beispiel 2 oral am 46. Tag nach der Infektion appliziert.

### Bestimmung der Wirksamkeit:

Die Selektion der Mäuse erfolgt am 54. Tag nach der Infektion. Die Auszählung der adulten Parasiten im Colon und Caecum wurde mikroskopisch durchgeführt. Der Behandlungserfolg in der Dosisgruppe wurde ins Verhältnis zur unbehandelten Kontrollgruppe gesetzt.

In der nachfolgenden Tabelle wird die Wirkung der Kombination gegen Heterakis spumosa in der Maus angegeben.

**Tabelle D Wirkung der Kombination von Imidacloprid und Ivermectin B₁ₐ/B_{1b} gegen Heterakis spumosa in der Maus nach oraler Applikation**

| **Wirkstoff und Menge [mg/kg]** | | **Reduktionsrate [%]** |
|---|---|---|
| Imidacloprid | 50,0 | 0 |
| Ivermectin B₁ₐ/B_{1b} | 0,1 | < 50 |
| Imidacloprid + Ivermectin B₁ₐ/B_{1b} | 50,0 | |
| | 0,1 | 100 |
| Imidacloprid | 25,0 | 0 |
| Imidacloprid + Ivermectin B₁ₐ/B_{1b} | 25,0 | |
| | 0,1 | 100 |
| Imidacloprid | 10,0 | 0 |
| Imidacloprid + Ivermectin B₁ₐ/B_{1b} | 10,0 | |
| | 0,1 | > 80 |
| Imidacloprid | 5,0 | 0 |
| Imidacloprid + Ivermectin B₁ₐ/B_{1b} | 5,0 | |
| | 0,1 | > 80 |

### Anwendungsbeispiel E

Die insektizide und nematozide Wirksamkeit von drei Imidacloprid/Ivermectin Formulierungen bei konstanten Applikationsvolumen von 0,1ml/kg wurde in vier Prüfgruppen am Hund vergleichend untersucht. Die Anwendung der Prüfsubstanzen erfolgte als Spot-On. In den Formulierungen betrug der Ivermectin-Anteil entsprechend 0,006 %, 0,05 % und 0,2 % Imidacloprid war in jeder der Testsubstanzen zu einem konstanten Anteil von 10 % enthalten. Alle Tiere der jeweiligen Behandlungs- und der Kontrollgruppen wurden in definierten Zeitabständen vor und nach der Behandlung einer klinischen Untersuchung auf Floh- und Nematodenbefall unterzogen.

| | |
|---|---|
| **Prüfzeitraum:** | 4 Wochen |

### Prüfsubstanzen:

### I. Imidacloprid

| | |
|---|---|
| Gehalt an a.i.: | 10 % GV |

### II. Ivermectin

| | |
|---|---|
| Gehalt an a.i.: | 0,006 % G/V (Beispiel E1) |
| | 0,05 % G/V (Beispiel E2) |
| | 0,2 % G/V (Beispiel E3) |

### Versuchstiere

| | |
|---|---|
| Spezies: | Hund *(Canis familiaris)* |
| Rasse: | Beagle |
| Anzahl: | 8 |
| Geschlecht: | 4 weibliche und 4 mannliche Tiere |
| Alter: | Welpen, 2-3 Monate alt |

### Experimentelle Infestation mit Flöhen

Jeder Hund wurde am Tag -3 vor der Behandlung mit ca. 100, bis zu vier Wochen alten Flöhen im Bereich der Schenkelinnenfalte infestiert. Die Reinfestationen fanden wöchentlich statt.

### Experimentelle Infestation mit Nematoden

20 Tage vor der Behandlung wurde alle Hunde mit jeweils 250 infektiösen Larven (1,3) von *Acylostoma caninum* infiziert.

### Applikation

Die Tiere wurden einmalig im Spot-On Verfahren behandelt. Jeweils 2 Tiere bildeten eine Behandlungsgruppe. Das Applikationsvolumen betrug für alle Tiere 0,1 ml/kg.

### Klinische Untersuchung der Wirksamkeit

Für die Beurteilung der insektiziden Wirksamkeit der Behandlung wurden alle Hunde vor den Behandlung und dann jeweils 24 Stunden nach der Behandlung bzw. nach jeder Flohreinfestation quantitativ auf Flohbefall untersucht. Die endoparasitizide Wirksamkeit wurde durch Auszählung der mit dem Kot ausgeschieden, abgetriebenen Würmer vor und nach der Behandlung (Tag 1-3 nach Behandlung) ermittelt.

### Ergebnisse

In allen Versuchsgruppen konnte eine insektizide Wirksamkeit von 100 % uber einen Zeitraum von 28 Tagen nachgewiesen werden. Die endoparasitizide Wirksamkeit ist dosierabhängig, siehe nachfolgende Tabelle.

| Formulierung (% Imidacloprid / % Ivermectin) | Wirksamkeit |
|---|---|
| 10/0,006 | 60 % |
| 10/0,05 | 95 % |
| 10/0,2 | 99 % |

## Patentansprüche

1. Verwendung von
(a) Avermectin B₁ₐ/B_{1b}, 22,23-Dihydroavermectin B₁ₐ/B_{1b}, Doramectin oder Moxidectin mit
(b) Imidacloprid, Ti 435 oder AKD 1022;
(c) gegebenenfalls weiteren Wirkstoffen sowie Verdünnungsmitteln oder Trägerstoffen
zur Herstellung von Mittel zur dermalen Verabreichung der unter a) und b) genannten Wirkstoffe zur Bekämpfung von Ekto- und Endoparasiten in einer einzigen Behandlung.

## Claims

1. Use of
(a) avermectin B₁ₐ/B_{1b}, 22,23-dihydroavermectin B₁ₐ/B_{1b}, doramectin or moxidectin with
(b) imidacloprid, Ti 435 or AKD 1022;
(c) optionally further active ingredients and diluents or carriers,
for the preparation of compositions for dermal application of the active ingredients mentioned under a) and b) for combatting of ecto- and endoparasites in a single treatment.

## Revendications

1. Utilisation de
(a) l'avermectine B₁ₐ/B_{1b}, la 22,23-dihydroavermectine B₁ₐ/B_{1b}, la doramectine ou la moxidectine avec
(b) de l'imidaclopride, Ti 435 ou AKD 1022;
(c) éventuellement d'autres matières actives et des diluants ou des supports,
pour la préparation des compositions pour l'administration dermique des matières actives mentionnées sous a) et b) destinées à combattre des ectoparasites et des endoparasites par un seul traitement.
